# EUROPEAN PATENT APPLICATION

(11) **EP 4 807 361 A1**
(43) Date of publication of application: **16.09.2026**
(21) Application number: 25209518.7
(22) Date of filing: 17.10.2025
(51) Int. Cl.: G01N 33/28, G01N 31/16

(54) **METHOD FOR DETERMINING THE WATER-SOLUBLE ACIDITY OF HYDROCARBON MIXTURES**

(30) Priority: 14.03.2025 BR 102025004931
(71) Applicant: Petroleo Brasileiro S.A. - PETROBRAS, 20031-912 Rio de Janeiro (BR)
(72) Inventor: CORTINAS ALBUQUERQUE, FLAVIO, 21941-915 Rio de Janeiro (BR); RIBEIRO DA SILVA, CLAUDIO ROBERTO, 21941-915 Rio de Janeiro (BR); SOUZA DE ALENCAR, MAURICIO, 21941-915 Rio de Janeiro (BR); SANGHIKIAN, NATHALIE, 21941-915 Rio de Janeiro (BR)
(74) Representative: Clarke, Modet y Cía., S.L.

(57) **Abstract**

The present invention relates to an analytical method for determining the acid content of an oil mixture that can be extracted into an aqueous phase at a given pH, that is, the water-soluble acidity of a mixture of hydrocarbons that can be extracted into an aqueous phase.

## Description

### FIELD OF APPLICATION

The present invention discloses an analytical method for determining the acid content that can be extracted by the aqueous phase of a hydrocarbon mixture. The present invention falls within the field of analytical chemistry, more specifically in the oil field, and can be applied for assessing the quality of fossil hydrocarbon mixtures that may be, or may become, intimately mixed with an aqueous phase.

### BACKGROUNDS OF THE INVENTION

Oil and other complex hydrocarbon mixtures may contain acidic components of different origins. Among the most common are the naphthenic acids, which are natural carboxylic acids found in oil and have biogenic origins. Short-chain carboxylic acids, such as acetic, formic, propionic, or glycolic acid, are also common, which acids can be of biogenic origin or be part of the formulation of various chemical agents added to the oil from production to refining.

Although more rarely, oils and its fractions may also contain inorganic acids, such as hydrochloric acid (HCl) and sulfuric acid (H₂SO₄), resulting from contamination by chemical agents used in the well stimulation, or may also be present during oil formation through mechanisms that are not fully understood. Such inorganic acids can be found in free form, dissolved in emulsified water droplets in the hydrocarbon mixture, or combined, forming organic salts with bases that are eventually present in the medium.

The presence of the acids of different origins in the oil is associated with several problems in the production and refining chain, such as the stabilization of emulsions with water and the promotion of corrosive processes. For this reason, when assessing the quality of the oil or its fractions, it is common to determine its total acidity, expressed as the amount of strong base, such as potassium hydroxide (KOH), for example, required to neutralize a given mass of sample. There are even standardized analytical methods for this purpose, such as the method described in standard ASTM D 664.

However, the total acidity of an oil is generally dominated by the naphthenic acids, which are almost always present in levels much higher than other acids. Because naphthenic acids are preferentially soluble in the hydrocarbon phase, methods for determining total acidity, such as ASTM D 664, are of little use when referring to the acidification of aqueous phases, as said acids preferentially remain in the oil phase. Therefore, a specific analytical method is required to evaluate only the acids that can be extracted into water.

Therefore, mixtures of hydrocarbons of fossil origin, such as oil and its fractions, may contain acids that, when in contact with water, are partially or completely extracted into the aqueous medium. Even if they are present in small amounts in the hydrocarbon mixture, depending on the volumetric ratio between the phases, these components can significantly acidify the aqueous medium, making it corrosive to metal surfaces.

US Patent 5,366,898 discloses a method for quantitatively determining the total base or acid equivalents of an oil, wherein the total base number of an oil sample of a measured volume is determined by solvating the oil sample, adding a predetermined amount of acidic species to the solvated sample to react the acidic species with any basic species contained in the oil sample, extracting the remaining acidic species into an extractant phase, separating the extractant phase from the oil sample, and measuring the acidic species content of the extractant phase to determine the amount of basic species in the oil sample. The same technique can be used to determine the total acid number of an oil sample by adding a predetermined amount of basic species to the sample and measuring the basic species content of the extractant phase.

In contrast, the present invention does not seek to determine the total acids of an oil or the pH of water with which it has been in contact. The present invention aims to determine the acid content of an oil capable of being extracted into an aqueous phase at a given pH. Furthermore, US Patent 5,366,898 discloses a method for determining the total acidity of an oil; however, the composition of oil contains many acidic components that will never migrate to an aqueous phase with which they come into contact, as they partition favorably into the organic medium.

US Patent 2,782,151 relates to a method for determining the hydrogen ion concentration of an oil sample that does not dissolve in water. The method involves mixing the oil sample with water and a small amount of a special wetting agent that promotes the mixing of water and oil. This creates a dispersion in which the oil is dispersed as small droplets in the water. Glass electrodes are then placed in the dispersion, and a potential difference develops between the electrodes based on the hydrogen ion concentration of the oil sample. This method allows the measurement of the acidity or alkalinity of the oil sample, which can be useful in various industries.

However, the present invention does not seek to determine the total acids of an oil or the pH of water with which it has been in contact. The present invention proposes to determine the acid content of an oil that can be extracted into an aqueous phase at a given pH. Furthermore, US Patent 2,782,151 teaches pH measurements of an aqueous phase that has been in contact with the oil, but does so after a single extraction step. When this is done, the acids present reach equilibrium with the water and its pH, so that not all acids are extracted. Multiple equilibration stages are necessary for all extractable acids to migrate into the water.

Patent document US20130309773 discloses a process for determining the total acid value (TAN) of an insulating mineral oil, wherein the method consists of the steps of placing an oil sample and a pre-established amount of a solution suitable for extracting the acidic fraction of the sample in a container; vigorously stirring the container's contents and waiting for the extractant phase to separate from the sample; the dosage in the extract phase with a KOH titration solution until an acid-base indicator present in the same changes color, and the TAN calculation based on the volume of the used titration solution according to the formula:$TAN \left(mg KOH/g oil\right) = \left(V\times 56.1\times M\right) / m$ where V is the volume of the titration solution used expressed in mL, 56.1 is the molecular weight of KOH, M is the titer of the KOH solution expressed in moles/liter, and m is the mass of the sample expressed in g.

However, the present invention does not aim to determine the total acids of an oil or the pH of water with which it has been in contact. The present invention proposes to determine the acid content of an oil capable of being extracted into an aqueous phase at a given pH. Furthermore, US patent document US20130309773 teaches pH measurements of an aqueous phase that has been in contact with the oil, but does so after a single extraction step. When this is done, the acids present reach equilibrium with the water and its pH, so that not all acids are extracted. Multiple equilibration stages are necessary for all extractable acids to migrate to the water.

As noted, the state of the art would benefit from an analytical method for determining the water-soluble acidity of hydrocarbon mixtures that, by enabling the measurement of the content of acidic species present in hydrocarbon mixtures and that are water-soluble, allows for the correct selection of the materials that may come into contact with the aqueous phase, or for the necessary corrosion prevention measures to be taken in advance. Furthermore, the method of the invention proposed herein allows for the determination of the acidity of the aqueous phase resulting from contact with the hydrocarbon phase, enabling the application of protective measures for workers exposed to these waters.

Furthermore, by enabling the measurement of the content of acidic species present in hydrocarbon mixtures and that are water-soluble, the present invention allows for the prediction of the deterioration to which lines and equipment are subject when in contact with these waters. And, by enabling the measurement of the acidic species present in hydrocarbon mixtures and that are water-soluble, the present invention allows for the proper treatment and disposal of these waters.

### BRIEF DESCRIPTION OF THE INVENTION

The present invention relates to an analytical method for determining the acid content of a mixture, that is, the water-soluble acidity of a mixture of hydrocarbons that can be extracted by an aqueous phase.

To implement the method, a known mass of the hydrocarbon mixture is placed in contact with water at a specific pH. A pH-sensitive electrode and a tube connected to a pump capable of adding a base solution of known concentration are immersed in the water layer. The pH electrode and the pump must be interconnected so that the latter is activated when the electrode detects a drop in pH. The oil and aqueous phases are stirred, causing acids to migrate from the former to the latter. The pH drop is detected by the electrode, which indicates the addition of base to bring the pH back to its initial value. This process occurs continuously until the pH value of the aqueous phase stops decreasing or after a defined period. At the end, the total volume of solution consumed is used to calculate the number of moles of added base, a value that is in turn divided by the mass of sample used in the test. Assuming that the reaction between base and acid is equimolar, the water-soluble acid content present in the hydrocarbon mixture is expressed as the number of millimoles of base per kilogram of the sample.

### BRIEF DESCRIPTION OF THE FIGURES

The subject matter of this invention will become fully clear in its technical aspects from the detailed description that will be provided based on the figures listed below, in which:

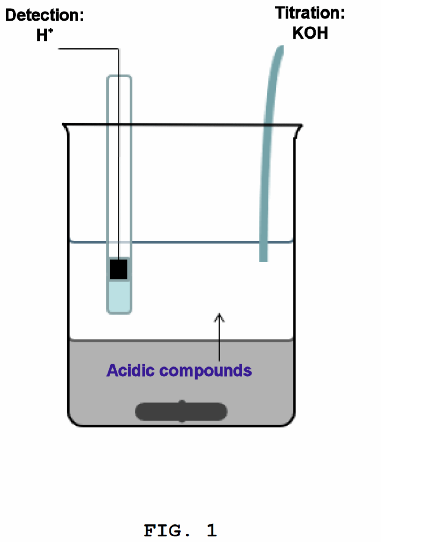

Figure 1 illustrates the soluble acidity determination scheme.

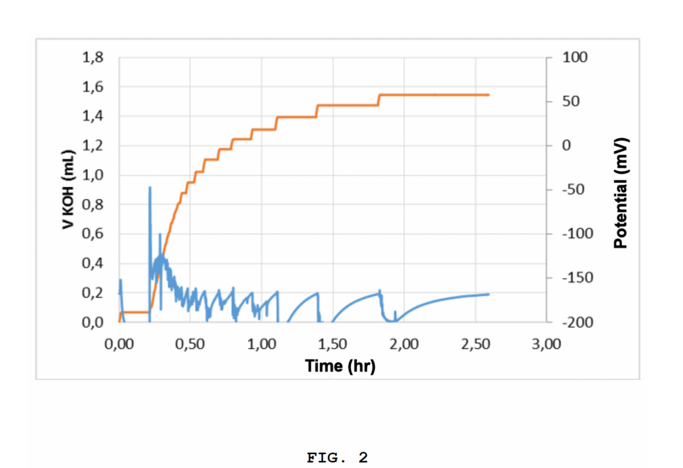

Figure 2 illustrates the base consumption graph.

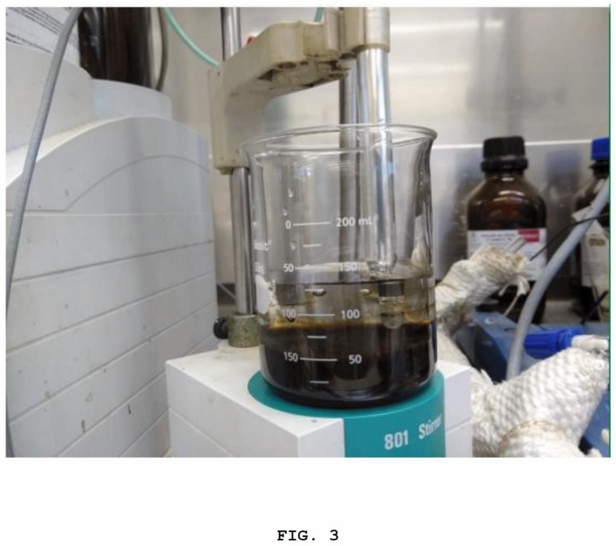

Figure 3 illustrates the image of the soluble acidity determination.

### DETAILED DESCRIPTION OF THE INVENTION

The present invention relates to an analytical method for determining the acid content of a mixture of hydrocarbons that can be extracted by an aqueous phase.

More specifically, the method of the present invention is carried out according to the following steps:
(i) In a 250 mL beaker containing a magnetic stir bar, 30 mL of a hydrocarbon solvent that is immiscible, denser, and non-reactive with water (organochlorine solvents, such as dichloromethane or chloroform, preferably dichloromethane) is dispensed;
(ii) Next, a glass funnel is placed inside the beaker, so that the end of its stem is immersed in the solvent and its neck rests on the side of the beaker;
(iii) Then, 100 mL of deionized water is added to the beaker (do not use a funnel for this purpose), wherein the volumetric ratio of solvent to water can vary between 1:10 and 100:10. Then, there is a two-phase liquid mixture inside the beaker, with the solvent in the lower phase and the water in the upper phase;
(iv) The beaker is then placed on a magnetic stirrer plate, without heating, and stirring is started as vigorously as possible, but without producing dispersion of one phase into the other; further, the interface between the two liquids must remain easily recognizable;
(v) Next, the sensitive end of a pH measuring electrode is immersed in the aqueous phase. The electrode must be coupled to a potentiometric titration system, with an aqueous solution of a strong base of known concentration as titrant: KOH or sodium hydroxide (NaOH), in concentrations between 0.001 and 0.5 mol/L, preferably 0.01 mol/L, depending on the content of water-soluble acids expected in the sample, if they were shown to be adequate; and
(vi) Next, the pH of the deionized water is set to the value at which the migration of soluble acids is desired; pH values between 4.5 and 7.5, preferably 6.0 and 7.0, are suitable. It may be necessary to add a small volume of strong acid diluted to 0.001-0.5 mol/L, preferably 0.01 mol/L, which is selected from the group of hydrochloric acid, sulfuric acid, or nitric acid, preferably nitric acid; or add a small volume of base to the aqueous phase to make this adjustment;
(vii) Once the reference pH has been adjusted, transfer between 5 and 20 g of the hydrocarbon mixture directly to the solvent phase, through the funnel, with the aid of 20 mL to 50 mL of the same solvent, that is, dichloromethane or chloroform, preferably dichloromethane;
(viii) Once the sample has been added, remove the funnel from the beaker and begin the potentiometric titration, so that the addition of the strong base KOH or NaOH, preferably KOH with a concentration between 0.001 and 0.5 mol/L, preferably 0.01 mol/L, occurs as soon as the pH drop is detected by the electrode, bringing the pH of the aqueous phase back to the reference value between 4.5 and 7.5, preferably 6.0 and 7.0. Prevent emulsion formation or drops of the oil phase from touching the electrode by reducing the intensity of the stirring until this ceases to occur. This preserves the correct mass transfer between the phases and the quality of the pH reading.

The process of transferring acidic compounds and correcting the pH to the reference value between 4.5 and 7.5, preferably between 6.0 and 7.0, by adding the strong base KOH or NaOH, preferably KOH with a concentration between 0.001 and 0.5 mol/L, preferably 0.01 mol/L, can take between 1 hour and 7 hours, and is completed when there is no more consumption of base solution (pH no longer moves from the reference value) or when a predefined period is reached. The water-soluble acid content of the hydrocarbon mixture, for the defined pH value of the aqueous phase, is calculated as follows:
- Soluble acid content; mmol/kg = [consumed volume of base; mL] * [base content of the solution; mol/L] / [sample mass; g] / 1000.

By detecting forms of acidity present in fossil hydrocarbons, the analytical method developed in this invention makes it possible to determine in advance the corrosive potential that water will have if it comes into contact with said hydrocarbons, since in both production environments and refineries, water that has been emulsified with oil must be processed and disposed of, passing through pipelines and metal equipment. It is necessary to know the acidity and corrosiveness that these waters may acquire after coming into contact with the hydrocarbon mixture, so that the correct selection of materials can be made and appropriate measures for corrosion protection can be taken in advance.

The method of the invention proposed herein is useful in both oil and natural gas exploration and production (E&P) and refining, since in both fields there are situations where oil and water are or are brought into contact, making it important to measure the degree of corrosivity that will be acquired by the latter. The invention finds application in assessing the quality of fossil hydrocarbon mixtures that may be, or may become, intimately mixed with an aqueous phase. It can also be applied to any situation in which it is necessary to determine the acidity that may be acquired by the aqueous stream after contact with hydrocarbon mixtures.

The following Example presents the results of the effectiveness test of the analytical method for determining the water-soluble acidity of hydrocarbon mixtures (Example 1).

### Example 1 - Effectiveness Test of the Analytical Method for Determining the Water-Soluble Acidity of Hydrocarbon Mixtures

To demonstrate the effectiveness of the analytical method, 300.11 g of an oil with API gravity = 20.7° was doped with 25.0 mg of 1-naphthylamine hydrochloride (C₁₀H₉N.HCl, molar mass = 179.646 g/mol).

Assuming that the HCl molecule present in the hydrochloride will dissociate and be extracted by water, this results in an oil containing 0.46 mmol/kg of acid capable of being extracted into an aqueous phase. Next, aliquots of approximately 10 g of doped and undoped oil were subjected to the method for determining the soluble acidity in water at pH 7.0. Water-soluble acidity contents of 0.56 mmol/kg and 0.15 mmol/kg were obtained. The difference between the acidity after and before doping, 0.41 mmol/kg, is very close to the acidity that was added to the oil.

Aliquots of oil doped and undoped with 1-naphthylamine hydrochloride were also analyzed according to the ASTM D 664 method, and total acidity numbers equal to 1.8 mg KOH/g were obtained in both. In other words, the method for determining total acidity is not capable of differentiating the aliquots based on the added acidity. If expressed as a total acidity number, the added soluble acidity is equivalent to 0.023 mg KOH/g, a difference imperceptible by the D 664 method, but significant by the method described in this invention.

Water-soluble acidity contents between 0.1 and 3.3 mmol/kg were determined in several oil samples, with API gravity ranging from 20 to 35°, for initial pH values ranging from 5.5 to 7.5.

It should be understood that the present invention is not limited to the details described herein and that the invention is capable of other embodiments and of being practiced or executed in a variety of ways, within the scope of the claims. Although specific terms have been used, such terms are to be interpreted in a generic and descriptive sense and not for the purpose of limitation.

## Claims

1. A METHOD FOR DETERMINING THE WATER-SOLUBLE ACIDITY OF HYDROCARBON MIXTURES, ***characterized in that*** it comprises the following steps:
(a) Adding 30 mL of dichloromethane or chloroform, preferably dichloromethane;
(b) Adding 100 mL of deionized water in step (a), wherein the volumetric ratio of solvent to water is 1:10 and 100:10;
(c) Stirring the mixture from the previous steps;
(d) Inserting a pH measuring electrode into the aqueous phase;
(e) Setting the pH value between 4.5 and 7.5, preferably 6.0 and 7.0, of the deionized water;
(f) Transferring 5 g to 20 g of the hydrocarbon mixture with 20 mL to 50 mL of dichloromethane or chloroform, preferably dichloromethane, to the solvent phase;
(g) Removing the funnel from the beaker;
(h) Measuring the pH;
(i) Reducing the stirring speed; and
(j) Stabilizing the pH at 4.5 and 7.5, preferably 6.0 and 7.0, after 1 hour and 7 hours.

2. THE METHOD according to claim 1*, **characterized in that*** in step (a) the solvent is dichloromethane.

3. THE METHOD according to claim 1*, **characterized in that*** in step (d) the electrode is coupled to a potentiometric titration system.

4. THE METHOD according to claim 1*, **characterized in that*** in step (e) the pH is adjusted to 4.5 and 7.5, preferably 6.0 and 7.0, with the addition of a strong acid or strong base.

5. THE METHOD according to claim 4, ***characterized in that** the* strong acid diluted to 0.001-0.5 mol/L, preferably 0.01 mol/L, is selected from the group of hydrochloric acid, sulfuric acid, and nitric acid, preferably nitric acid.

6. THE METHOD according to claim 5, ***characterized in that*** in step (f) the transfer is performed with a glass funnel.

7. THE METHOD according to claim 1, ***characterized in that*** in step (h) a strong base is added.

8. THE METHOD according to claim 7, ***characterized in that*** the strong base is KOH or NaOH, preferably KOH, with a concentration between 0.001 and 0.5 mol/L, preferably 0.01 mol/L.

9. THE METHOD according to claim 1, ***characterized in that*** in step (j), the pH is stabilized to adjust it to a value between 4.5 and 7.5, preferably 6.0 and 7.0, by gradually adding the base, said base being at a concentration of 0.001 and 0.5 mol/L, preferably 0.010 mol/L, and said addition being performed over a period of time between 1 hour and 7 hours.
